# EUROPEAN PATENT APPLICATION

(11) **EP 2 676 660 A1**
(43) Date of publication of application: **25.12.2013**
(21) Application number: 13173190.3
(22) Date of filing: 21.06.2013
(51) Int. Cl.: A61K 9/16, A61K 9/20, A61K 9/24, A61K 31/19, A61K 31/196, A61K 31/44

(54) **Compositions preventing hypertension comprising soluplus**

(30) Priority: 22.06.2012 TR 201207280
(71) Applicant: Sanovel Ilac Sanayi ve Ticaret A.S., 34460 Istanbul (TR)
(72) Inventor: Cifter, Ümit, 34460 Istanbul (TR); Türkyilmaz, Ali, 34460 Istanbul (TR); Mutlu, Onur, 34460 Istanbul (TR)
(74) Representative: Sevinç, Erkan

(57) **Abstract**

The invention is related to a pharmaceutical composition, characterized in that; it comprises angiotensin II receptor antagonist or a pharmaceutically acceptable salt or ester or racemic mixture thereof with polyvinyl caprolactam- polyvinyl acetate-polyethylene glycol graft copolymer.

## Description

### Technical Field

The invention is relates to angiotensin II receptor antagonists. More specifically, this invention is relates to stable compositions obtained by adding calcium channel blocker or thiazide or both of them to the angiotensin II receptor antagonist.

### Background of the Invention

Valsartan is a specific angiotensin II receptor antagonist that selectively and competatively blocks the AT1-receptors that mediate the effects of angiotensin II related with vasopressor and aldosterone. Its chemical name is N-(p-(o-1H-Tetrazol-5-ilfenil)benzil)-N-valeril-L-valin and its chemical structure is shown in the Formula I.

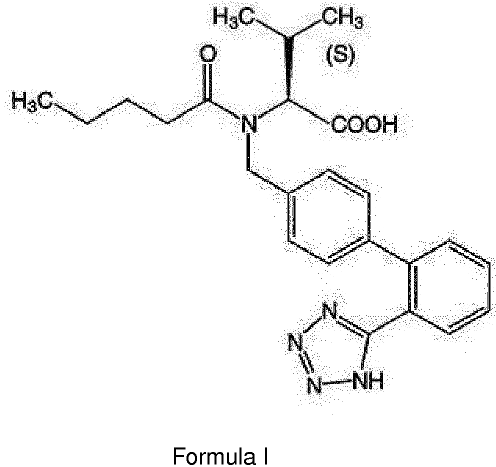

Valsartan molecule is used in hypertension theraphy. 80, 160 and 320 mg tablets of valsartan are present currently and they are administered orally.

Azilsartan which has a chemical name as 2-ethoxy-1-((2'-(5-oxo-2,5-dihydro-1,2,4-oxadiazol-3yl)-biphenyl-4-yl)methyl)-1H-benzimidazole-7-carboxylic acid (hereinafter referred as "Compound I") is a novel angiotensin II receptor antagonist and its chemical structure is shown in the Formula 2.

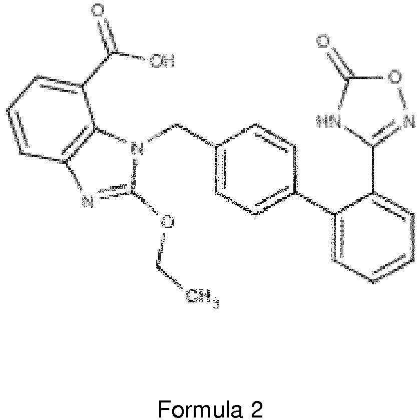

Amlodipine is a calcium channel blocker administered orally. Its chemical name is (RS)-3-Ethyl-5-methyl-2-(2-aminoetoxymethyl)-4-(2-chlorophenyl)-6-methyl-1,4-dihydropiridine-3,5-dicarboxylate and its chemical structure is shown in the Formula 3.

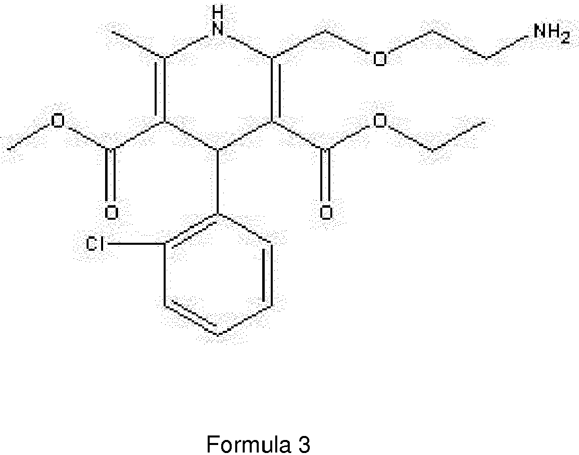

5 to 10 mg tablets of amlodipine are present currently and they are administered orally.

Hydrochlorothiazide is a diuretic with a thiazide structure and it is used in the treatment of oedema and hypertension. Its chemical name is 6-Chlorine-3,4-dihydro-2H-1,2,4-benzothiyadiazine-7-sulphonamide-1,1-dioxide and its chemical structure is shown in the Formula 4.

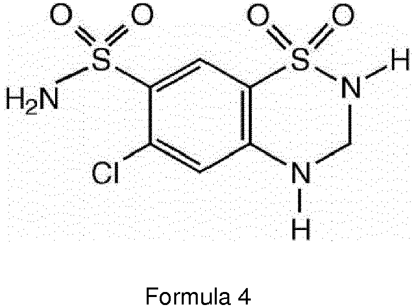

There are various applications in the patent literature related to the combination of valsartan, amlodipine, hydrochlorothiazide.

Patent application no WO2007022113 describes the solid composition composed of valsartan, amlodipine and excipient.

Patent application no WO2011001440 describes the composition composed of valsartan and mannitol with povidone. Additionally, hydrochloro thiazide or amlodipine may also be added to this formulation.

Patent application no US20110028456 claims a composition, which is formed by a group comprising valsartan and amlodipine with a water soluble or insoluble polymer.

Patent application no WO03097045 describes the kit composition composed of valsartan, hydrochloro thiazide and amlodipine.

Patent application no WO 2010081824 describes the composition comprising the group in which valsartan is selected with enantiometrically pure (S).

Patent application no WO2009022169 describes the solid pharmaceutical composition comprising core. The core comprises granules prepared by wet granulation and the granules comprise valsartan and at least one excipient. It is characterized in that, its core contains 3% or less moisture. In the dependant claims, it is indicated that additionally it comprises another active agent and amlodipine and hydrochlorothiazide molecules are exemplified.

The patent application no WO2009042960 describes the pharmaceutical distribution package containing fixed single dosage which is combined in single distribution package and seperated from each other within the package. It comprises a core capsule containing the first pharmaceutical formulation surrounded by the semi capsule which comprises the second pharmaceutical formulation. Valsartan, amlodipine and diuretic molecules are present in the active agent group.

Patent application no WO2009048848 describes the pharmaceutical composition in suspension form, administered orally and comprising valsartan and glycerol or syrup or mixture thereof, at least one or two or more substances selected among preservative, buffer system and suspending/stabilizing agent. With the dependant claim, it is indicated that it additionally comprises a second active agent, and the hydrochlorothiazide and calcium channel blockers are explained.

The solubility and dissolution rate of the active agents used in the formulations directly affect the bioavailability. Therefore, it is very important to increase the solubility and the dissolution rate of the said active agents.

Another problem related to these active agents is the stability problems experienced with the effect of environmental and physical conditions as it also the case in many active agents. Angiotensin II receptor antagonists calcium channel blockers and thiazide diuretics are the active agents which are effected from temperature, air and humidity conditions very much. Exposure to air and humidity causes structural degradation and chemical behaviour changes in the said active agents. The stability of the products developed is not at a desired level and the shelf life thereof is shortened. Additionally, these active agents may react with the excipients which are used together in the development step of the formulation. This situation leads to impurity in the formulations and participation of unwanted compositions into the formulations.

It is also known in the pharmaceutical field that, when formulating low dose pharmaceutical active ingredients for administration to those in need of therapy, it is desired to ensure an even dispersion of the pharmaceutically active ingredients with the pharmaceutical excipients for obtaining a proper dosage and homogeneity. Thus, each dosage form contains the desired amount of active agent. Therefore it would be desirable to provide improved processes for preparing solid oral dosage forms that have high content uniformity and that disperse well upon oral administration.

Consequently, a novelty is required in the art of formulations used for preventing and treating hypertension.

### Object and Brief Description of the Invention

The present invention is a composition of angiotensin II receptor antagonist and/or calcium channel blocker and/or thiazide diuretic which is easily applicable and which eliminates all the disadvantages mentioned above and provides additional advantages in the related technical field.

Based on the prior art, the main object of the invention is to obtain an effective and stable composition that can be used in hypertension disease.

A further object of the invention is to obtain angiotensin II receptor antagonist and/or calcium channel blocker and/or thiazide composition which has a desired solubility and dissolution rates.

A further object of the invention is to obtain angiotensin II receptor antagonist and/or calcium channel blocker and/or thiazide composition which has a content uniformity at a desired level.

Another object of the invention is to facilitate the production process by obtaining granules which do not adhere onto the machine and equipments during the production and which has superior flow property.

A further object of the invention is to obtain the angiotensin II receptor antagonist composition.

A further object of the invention is to obtain the angiotensin II receptor antagonist and calcium channel blocker composition.

A further object of the invention is to obtain angiotensin II receptor antagonist and/or calcium channel blocker and/or thiazide composition.

A further object of the invention is to obtain angiotensin II receptor antagonist and/or calcium channel blocker and/or thiazide composition which has bioavailability values at the desired level.

A pharmaceutical composition is developed in order to achieve all the objects which are mentioned above and which can be understood from the detailed description below.
In a preferred embodiment of the present invention, the said novelty is realized by the angiotensin II receptor antagonist and polyvinyl caprolactam- polyvinyl acetate-polyethylene glycol graft copolymer. The said angiotensin II receptor antagonist is selected among valsartan, losartan, azilsartan, irbesartan, telmisartan, candesartan, eprosartan, olmesartan medoxomil. The said angiotension II receptor antagonist preferably comprises valsartan or a pharmaceutically acceptable salt or ester or racemic mixture thereof.

A preferred embodiment of the invention additionally comprises a calcium channel blocker selected from amlodipine, felodipine, azelnidipine, bepridil, diltiazem, niphedipin, verapamil. The said calcium channel blocker comprises amlodipine or a pharmaceutically acceptable salt or ester or racemic mixture thereof.

A preferred embodiment of the invention additionally comprises a thiazide diuretic selected among hydrochlorothiazide, chlorinetalidone, indapamide, flupamide. The said thiazide diuretic comprises hydrochlorothiazide or a pharmaceutically acceptable salt or ester or racemic mixture thereof.

In a preferred embodiment of the invention, the said composition is tablet. Preferably the tablet is compressed with a pressure of 20 to 50 kN, preferably 25 to 45 kN, and more preferably 30 to 37 kN. The dissolution time of the composition shortens and the dissolution values increase to the upper level thanks to these preferred compression force intervals.

In a preferred embodiment of the invention, the said composition additionally comprises at least one excipient.

In another preferred embodiment of the invention, the said excipient is at least one of the substances selected from the group consisting of diluents, binders, plasticizers, disintegrants, glidants, lubricants or a mixture of them.

In another preferred embodiment of the invention, the said diluents are at least one of the substances selected from the group consisting of dextrose, magnesium carbonate, magnesium oxide, sucrose, cellulose acetate, maltodextrine, lactose, microcrystalline cellulose, starch, mannitole, calcium phosphate anhydrate, calcium phosphate dihydrate, calcium phosphate trihydrate, dibasic calcium phosphate and glucose or a mixture of them.

In another preferred embodiment of the invention, the said binders are at least one of the substances selected from the group consisting of hydroxyethyl cellulose (HEC), chitosan, carbomer, Carragen, pectin, collagen, guar gum, gum tragacanth, shellac, carnauba wax, cetyl alcohol, stearic acid, polyamide, hydrogenated castor oil, alginic acid, beeswax, methacrylic acid/ethyl acrylate copolymer, dimethyl amino ethyl methacrylate/butyl methacrylate/metal methacrylate copolymer, hydroxypropyl methyl cellulose acetate succinate, gelatine, derivatives of methacrylate, paraffine, polyvinyl acetate, cellulose acetate, cellulose phthalate, cellulose propionate, cellulose butyrate, setostearyl alcohol, polimethacrylate, glyceryl behenate, polyviniylpyrolidone (povidone), cellulose derivatives such as hydroxypropyl methyl cellulose (HPMC), hydroxypropyl cellulose (HPC), carboxy methyl cellulose (CMC), methyl cellulose (MC), ethyl cellulose, polyethylene oxide, gelatine or a mixture of them.

In another preferred embodiment of the invention, the said plasticizer are at least one of the substances selected from the group consisting of castor oil, glycerin, citrate esters (acetyl tri-n-butyl citrate, acetyl triethyl citrate, tri-n-butyl citrate, triethyl citrate) dibutyl sebacate, triacetin, diethyl phthalate, low molecular weight polyethylene glycols or a mixture of them in suitable amounts.

In another preferred embodiment of the invention, the said disintegrants are at least one of the substances selected from the group consisting of crosscarmellose sodium, sodium starch glycolate, crospovidone, starch (e.g. corn, potato), alginic acid or a mixture of them.

In another preferred embodiment of the invention, the said glidant is at least one of the substances selected from the group consisting of colloidal silicone dioxide, talc, aluminium silicate, magnesium silicate or a mixture of them.

In another preferred embodiment of the invention, the said lubricant is at least one of the substances selected from the group consisting of glyceryl behenate, glyceryl monostearate calcium stearate, magnesium stearate, mineral oil, sodium stearyl fumarate, talc, polyethylene glycol, stearic acid or a mixture of them.

Another preferred embodiment of the invention is a method for preparing the said pharmaceutical combination which comprises the following steps:
a. adding colloidal silicone dioxide to valsartan alone or mixture of valsartan and amlodipine besylate or valsartan, amlodipine besylate and hydrochlorothiazide colloidal silicone dioxide and mixing them together by sieving,
b. adding polyvinyl caprolactam-polyvinyl acetate-polyethylene glycol graft copolymer, crosscarmellose sodium and mannitol to this mixture and blending it,
c. afterwards, adding magnesium stearate to this mixture and blending them,
d. compressing the last mixture in the tablet machine or filling it into the capsule,
e. coating the tablet with a coating material.

Another preferred embodiment of the invention is a method for preparing the said pharmaceutical combination which comprises the following steps:
a. adding colloidal silicone dioxide to valsartan alone or mixture of valsartan and amlodipine besylate or valsartan, amlodipine besylate and hydrochlorothiazide colloidal silicone dioxide and mixing them together by sieving,
b. adding crosscarmellose sodium and mannitole to this mixture and blending them,
c. granulating, drying and sieving with the addition of alcoholic/hydroalcoholic solution of polyvinyl caprolactam-polyvinyl acetate-polyethylene glycol graft copolymer to the mixture formed,
d. afterwards, adding magnesium stearate to this mixture and blending them,
e. compressing the last mixture in the tablet machine or filling it into the capsule,
f. coating the tablet with a coating material.

### Detailed Description of the Invention

### Examples - 1

### A. DIRECT COMPRESSION

### a.

| **Ingredients** | **% Weight** |
|---|---|
| ***Core tablet*** | |
| Valsartan | 5-40 |
| Polyvinyl caprolactam-polyvinyl acetate-polyethylene glycol graft | 5-70 |
| Mannitol or dibasic calcium phosphate | 5-70 |
| Crosscarmellose sodium or Sodium starch glycolate | 0.5-10 |
| Colloidal silicon dioxide | 0.25-2 |
| Magnesium stearate or Sodium stearyl fumarate | 0.25-2 |

| ***Film coating*** | |
|---|---|
| Opadry AMB or Collicoat IR | 1-5 |

Production Method: Valsartan and colloidal silicone dioxide are sieved and mixed together (co-shifting). Afterwards, polyvinyl caprolactam-polyvinyl acetate-polyethylene glycol graft copolymer, crosscarmellose sodium and mannitol are added to this mixture and blended. Afterwards, this mixture is blended with magnesium stearate and compressed in the tablet machine or may be filled into capsules. The coating process is realized with a coating material such as Opadry AMB or Collicoat IR having moisture barrier.

### b.

| **Ingredients** | **% Weight** |
|---|---|
| ***Core tablet*** | |
| Valsartan | 5-40 |
| Amlodipin besilat | 0.3-1.3 |
| Polyvinyl caprolactam-polyvinyl acetate-polyethylene glycol graft | 5-65 |
| Mannitol or dibasic calcium phosphate | 5-65 |
| Crosscarmellose sodium or Sodium starch glycolate | 0.5-10 |
| Colloidal silicon dioxide | 0.25-2 |
| Magnesium stearate or Sodium stearyl fumarate | 0.25-2 |

| ***Film coating*** | |
|---|---|
| Opadry AMB or Collicoat IR | 1-5 |

Production Method: Valsartan and amlodipine besilate is sieved with colloidal silicone dioxide and mixed (co-shifting). Afterwards, polyvinyl caprolactam-polyvinyl acetate-polyethylene glycol graft copolymer, crosscarmellose sodium and mannitol are added to this mixture and blended. Afterwards, this mixture is blended with magnesium stearate and compressed in the tablet machine or may be filled into capsules. The coating process is realized with a coating material such as Opadry AMB or Collicoat IR having moisture barrier.

### c.

| **Ingredients** | **% Weight** |
|---|---|
| ***Core tablet*** | |
| Valsartan | 5-40 |
| Amlodipin Besilat | 0.3-1.3 |
| Hydrochlorothiazide | 0.5-3.5 |
| Polyvinyl caprolactam-polyvinyl acetate-polyethylene glycol graft | 5-60 |
| Mannitol or dibasic calcium phosphate | 5-60 |
| Crosscarmellose sodium or Sodium starch glycolate | 0.5-10 |
| Colloidal silicon dioxide | 0.25-2 |
| Magnesium stearate or Sodium stearyl fumarate | 0.25-2 |

| ***Film coating*** | |
|---|---|
| Opadry AMB or Collicoat IR | 1-5 |

**Production Method:** Valsartan, amlodipine besilate and hydrochlorothiazide are sieved with colloidal silicone dioxide and mixed (co-shifting). Afterwards, polyvinyl caprolactam-polyvinyl acetate-polyethylene glycol graft copolymer, crosscarmellose sodium and mannitol are added to this mixture and blended. Afterwards, this mixture is blended with magnesium stearate and compressed in the tablet machine or may be filled into capsules. The coating process is realized with a coating material such as Opadry AMB or Collicoat IR having moisture barrier.

*Co-shifting:* When the active agents and silicone dioxide are added by being sieved together, the fluidity of the powders improves.

### d.

| **Ingredients** | **% Weight (w/w)** |
|---|---|
| **Core tablet** | |
| *1st layer* | |
| Valsartan | 5-40 |
| Polyvinyl caprolactam-polyvinyl acetate-polyethylene glycol graft | 5-30 |
| Mannitol or dibasic calcium phosphate | 2.5-30 |
| Crosscarmellose sodium or Sodium starch glycolate | 0.5-10 |
| Colloidal silicon dioxide | 0.125-1.5 |
| Magnesium stearate or Sodium stearyl fumarate | 0.125-2.0 |

| *2nd Layer* | |
|---|---|
| Amlodipin Besilat | 0.3-1.3 |
| Hydrochlorothiazide | 0.5-3.5 |
| Polyvinyl caprolactam-polyvinyl acetate-polyethylene glycol graft | 5-30 |
| Mannitol or dibasic calcium phosphate | 2.5-30 |
| Colloidal silicon dioxide | 0.125-1.5 |
| Magnesium stearate or Sodium stearyl fumarate | 0.125-2.0 |

| **Film coating** | |
|---|---|
| Opadry AMB or Collicoat IR | 1-5 |

**Production Method:** In the 1^{st} layer valsartan is sieved with colloidal silicone dioxide and mixed (co-shifting). Afterwards, polyvinyl caprolactam-polyvinyl acetate-polyethylene glycol graft copolymer, crosscarmellose sodium and mannitol are added to this mixture and blended. Afterwards, this mixture is blended with magnesium stearate. In the 2^{nd} layer, in a different mixer, amlodipine besilate and hydrochlorothiazide is sieved with colloidal silicone dioxide and mixed (co-shifting). Afterwards, polyvinyl caprolactam-polyvinyl acetate-polyethylene glycol graft copolymer and mannitol are added to this mixture and blended. Afterwards, this mixture is is blended with magnesium stearate. Afterwards, powders prepared for two layers are compressed in the bi layer tablet machine or may be filled into capsules. The coating process is realized with a coating material such as Opadry AMB or Collicoat IR having moisture barrier.

### e.

| **Ingredients** | **% Weight (w/w)** |
|---|---|
| **Core tablet** | |
| *1st Layer* | |
| Valsartan | 5-40 |
| Polyvinyl caprolactam-polyvinyl acetate-polyethylene glycol graft | 3-20 |
| Crosscarmellose sodium or Sodium starch glycolate | 0.25-5 |
| Mannitol or dibasic calcium phosphate | 1-20 |
| Colloidal silicon dioxide | 0.1-1 |
| Magnesium stearate or Sodium stearyl fumarate | 0.1-0.5 |

| *2nd layer* | |
|---|---|
| Amlodipin Besilat | 0.3-1.3 |
| Crosscarmellose sodium or Sodium starch glycolate | 0.25-5 |
| Polyvinyl caprolactam-polyvinyl acetate-polyethylene glycol graft | 3-20 |
| Mannitol or dibasic calcium phosphate | 1-20 |
| Colloidal silicon dioxide | 0.1-1 |
| Magnesium stearate or Sodium stearyl fumarate | 0.1-0.5 |

| *3rd Layer* | |
|---|---|
| Hydrochlorothiazide | 0.5-3.5 |
| Polyvinyl caprolactam-polyvinyl acetate-polyethylene glycol graft | 3-20 |
| Mannitol or dibasic calcium phosphate | 1-20 |
| Colloidal silicon dioxide | 0.1-1 |
| Magnesium stearate or Sodium stearyl fumarate | 0.1-0.5 |

| **Film coating** | |
|---|---|
| Opadry AMB or Collicoat IR | 1-5 |

**Production Method:** In the 1^{st} layer valsartan is sieved with colloidal silicone dioxide and mixed (co-shifting). Afterwards, polyvinyl caprolactam-polyvinyl acetate-polyethylene glycol graft copolymer, crosscarmellose sodium and mannitol are added to this mixture and blended. Afterwards, this mixture is blended with magnesium stearate. In the 2^{nd} layer, in a different mixer, amlodipine besilate is sieved with colloidal silicone dioxide and mixed (co-shifting). Afterwards, polyvinyl caprolactam-polyvinyl acetate-polyethylene glycol graft copolymer, crosscarmellose sodium and mannitol are added to this mixture and blended. Afterwards, this mixture is is blended with magnesium stearate. In the 3^{rd} layer, in a different mixer, hydrochlorothiazide is sieved with colloidal silicone dioxide and mixed (co-shifting). Afterwards, polyvinyl caprolactam-polyvinyl acetate-polyethylene glycol graft copolymer and mannitol are added to this mixture and blended. Afterwards, this mixture is is blended with magnesium stearate. Afterwards, powders prepared for three layers are compressed in the tri layer tablet machine or may be filled into capsules. The coating process is realized with a coating material such as Opadry AMB or Collicoat IR having moisture barrier.

### B. WET GRANULATION

### a.

| **Ingredients** | **% Weight** |
|---|---|
| ***Core tablet*** | |
| Valsartan | 5-40 |
| Polyvinyl caprolactam-polyvinyl acetate-polyethylene glycol graft | 5-70 |
| Mannitol or dibasic calcium phosphate | 5-70 |
| Crosscarmellose sodium or Sodium starch glycolate | 0.5-10 |
| Colloidal silicon dioxide | 0.25-2 |
| Magnesium stearate or Sodium stearyl fumarate | 0.25-2 |
| ***Film coating*** | |
| Opadry AMB or Collicoat IR | 1-5 |

Production Method: Valsartan is sieved with colloidal silicone dioxide and mixed (co-shifting). Afterwards, crosscarmellose sodium and mannitole are added to this mixture and blended. Afterwards, this mixture is granulated, dried and sieved with the alcoholic/hydroalcoholic solution of polyvinyl caprolactam-polyvinyl acetate-polyethylene glycol graft copolymer. Afterwards, this mixture is blended with magnesium stearate and compressed in the tablet machine or may be filled into capsules. The coating process is realized with a coating material such as Opadry AMB or Collicoat IR having moisture barrier.

### b.

| **Ingredients** | **% Weight** |
|---|---|
| ***Core tablet*** | |
| Valsartan | 5-40 |
| Amlodipin Besilat | 0.3-1.3 |
| Polyvinyl caprolactam-polyvinyl acetate-polyethylene glycol graft | 5-65 |
| Mannitol or dibasic calcium phosphate | 5-65 |
| Crosscarmellose sodium or Sodium starch glycolate | 0.5-10 |
| Colloidal silicon dioxide | 0.25-2 |
| Magnesium stearate or Sodium stearyl fumarate | 0.25-2 |

| ***Film coating*** | |
|---|---|
| Opadry AMB or Collicoat IR | 1-5 |

Production Method: Valsartan and amlodipine besilate is sieved with colloidal silicone dioxide and mixed (co-shifting). Afterwards, crosscarmellose sodium and mannitole are added to this mixture and blended. Afterwards, this mixture is granulated, dried and sieved with the alcoholic/hydroalcoholic solution of polyvinyl caprolactam-polyvinyl acetate-polyethylene glycol graft copolymer. Afterwards, this mixture is blended with magnesium stearate and compressed in the tablet machine or may be filled into capsules. The coating process is realized with a coating material such as Opadry AMB or Collicoat IR having moisture barrier.

### c.

| **Ingredients** | **% Weight** |
|---|---|
| ***Core tablet*** | |
| Valsartan | 5-40 |
| Amlodipin Besilat | 0.3-1.3 |
| Hydrochlorothiazide | 0.5-3.5 |
| Polyvinyl caprolactam-polyvinyl acetate-polyethylene glycol graft | 5-60 |
| Mannitol or dibasic calcium phosphate | 5-60 |
| Crosscarmellose sodium or Sodium starch glycolate | 0.5-10 |
| Colloidal silicon dioxide | 0.25-2 |
| Magnesium stearate or Sodium stearyl fumarate | 0.25-2 |

| ***Film coating*** | |
|---|---|
| Opadry AMB or Collicoat IR | 1-5 |

**Production Method:** Valsartan, amlodipine besilate and hydrochlorothiazide are sieved with colloidal silicone dioxide and mixed (co-shifting). Afterwards, crosscarmellose sodium and mannitole are added to this mixture and blended. Afterwards, this mixture is granulated, dried and sieved with the alcoholic/hydroalcoholic solution of polyvinyl caprolactam-polyvinyl acetate-polyethylene glycol graft copolymer. Afterwards, this mixture is blended with magnesium stearate and compressed in the tablet machine or may be filled into capsules. The coating process is realized with a coating material such as Opadry AMB or Collicoat IR having moisture barrier.

### d.

| **Ingredients** | **% Weight (w/w)** |
|---|---|
| **Core tablet** | |
| *1^{st} Layer* | |
| Valsartan | 5-40 |
| Polyvinyl caprolactam-polyvinyl acetate-polyethylene glycol graft | 5-30 |
| Mannitol or dibasic calcium phosphate | 2.5-30 |
| Crosscarmellose sodium or Sodium starch glycolate | 0.5-10 |
| Colloidal silicon dioxide | 0.125-1.5 |
| Magnesium stearate or Sodium stearyl fumarate | 0.125-0.75 |

| *2nd Layer* | |
|---|---|
| Amlodipin Besilat | 0.3-1.3 |
| Hydrochlorothiazide | 0.5-3.5 |
| Polyvinyl caprolactam-polyvinyl acetate-polyethylene glycol graft | 5-30 |
| Mannitol or dibasic calcium phosphate | 2.5-30 |
| Colloidal silicon dioxide | 0.125-2.0 |
| Magnesium stearate or Sodium stearyl fumarate | 0.125-2.0 |

| **Film coating** | |
|---|---|
| Opadry AMB or Collicoat IR | 1-5 |

**Production Method:** In the 1^{st} layer valsartan is sieved with colloidal silicone dioxide and mixed (co-shifting). Afterwards, crosscarmellose sodium and mannitole are added to this mixture and blended. Afterwards, this mixture is granulated, dried and sieved with the alcoholic/hydroalcoholic solution of polyvinyl caprolactam-polyvinyl acetate-polyethylene glycol graft copolymer. Afterwards, it is mixed with magnesium stearate. In the 2^{nd} layer, in a different mixer, amlodipine besilate and hydrochlorothiazide is sieved with colloidal silicone dioxide and mixed (co-shifting). Afterwards, mannitole is added to this mixture and blended. Afterwards, this mixture is granulated, dried and sieved with the alcoholic/hydroalcoholic solution of polyvinyl caprolactam-polyvinyl acetate-polyethylene glycol graft copolymer. Afterwards, it is mixed with magnesium stearate. Afterwards, powders prepared for two layers are compressed in the bi layer tablet machine or may be filled into capsules. The coating process is realized with a coating material such as Opadry AMB or Collicoat IR having moisture barrier.

### e.

| **Ingredients** | **% Weight (w/w)** |
|---|---|
| **Core tablet** | |
| *1st Layer* | |
| Valsartan | 5-40 |
| Polyvinyl caprolactam-polyvinyl acetate-polyethylene glycol graft | 3-20 |
| Crosscarmellose sodium or Sodium starch glycolate | 0.25-5 |
| Mannitol or dibasic calcium phosphate | 1-20 |
| Colloidal silicon dioxide | 0.1-1 |
| Magnesium stearate or Sodium stearyl fumarate | 0.1-0.5 |

| *2^{nd} Layer* | |
|---|---|
| Amlodipin Besilat | 0.3-1.3 |
| Crosscarmellose sodium or Sodium starch glycolate | 0.25-5 |
| Polyvinyl caprolactam-polyvinyl acetate-polyethylene glycol graft | 3-20 |
| Mannitol or dibasic calcium phosphate | 1-20 |
| Colloidal silicon dioxide | 0.1-1 |
| Magnesium stearate or Sodium stearyl fumarate | 0.1-0.5 |
| *3rd Layer* | |
| Hydrochlorothiazide | 0.5-3.5 |
| Polyvinyl caprolactam-polyvinyl acetate-polyethylene glycol graft | 3-20 |
| Mannitol or dibasic calcium phosphate | 1-20 |
| Colloidal silicon dioxide | 0.1-1 |
| Magnesium stearate or Sodium stearyl fumarate | 0.1-0.5 |

| **Film coating** | |
|---|---|
| Opadry AMB or Collicoat IR | 1-5 |

**Production Method:** In the 1^{st} layer valsartan is sieved with colloidal silicone dioxide and mixed (co-shifting). Afterwards, crosscarmellose sodium and mannitole are added to this mixture and blended and this mixture is granulated, dried and sieved with the alcoholic/hydroalcoholic solution of polyvinyl caprolactam-polyvinyl acetate-polyethylene glycol graft copolymer. Afterwards, the mixture is blended with magnesium stearate.

In the 2^{nd} layer, in a different mixer, amlodipine besilate is sieved with colloidal silicone dioxide and mixed (co-shifting). Afterwards, crosscarmellose sodium and mannitole are added to this mixture and blended. The mixture is granulated, dried and sieved with the alcoholic/hydroalcoholic solution of polyvinyl caprolactam-polyvinyl acetate-polyethylene glycol graft copolymer. Afterwards, the mixture is blended with magnesium stearate.

In the 3^{rd} layer, in a different mixer, hydrochlorothiazide is sieved with colloidal silicone dioxide and mixed (co-shifting). Afterwards, mannitole is added to this mixture and blended and this mixture is granulated, dried and sieved with the alcoholic/hydroalcoholic solution of polyvinyl caprolactam-polyvinyl acetate-polyethylene glycol graft copolymer. Afterwards, the mixture is blended with magnesium stearate.
Afterwards, powders prepared for three layers are compressed in the tri layer tablet machine or may be filled into capsules. The coating process is realized with a coating material such as Opadry AMB or Collicoat IR having moisture barrier.

### C. HOT MELTING

### a.

| **Ingredients** | **% Weight** |
|---|---|
| ***Core tablet*** | |
| Valsartan | 5-40 |
| Polyvinyl caprolactam-polyvinyl acetate-polyethylene glycol graft | 5-70 |
| Mannitol or dibasic calcium phosphate | 5-70 |
| Crosscarmellose sodium or Sodium starch glycolate | 0.5-10 |
| Colloidal silicon dioxide | 0.25-2 |
| Magnesium stearate or Sodium stearyl fumarate | 0.25-2 |

| ***Film coating*** | |
|---|---|
| Opadry AMB or Collicoat IR | 1-5 |

Production Method: Valsartan, polyvinyl caprolactam- polyvinyl acetate-polyethylene glycol graft copolymer, mannitole and crosscarmellose sodium are mixed, extruded by being melted, cooled and sieved. Then, colloidal silicone dioxide is added to the obtained granule. Finally, magnesium stearate is added and mixed. Compression is applied in the tablet machine or filled into capsule. The coating process is realized with a coating material such as Opadry AMB or Collicoat IR having moisture barrier.

### b.

| **Ingredients** | **% Weight** |
|---|---|
| ***Core tablet*** | |
| Valsartan | 5-40 |
| Amlodipine Besylate | 0.3-1.3 |
| Polyvinyl caprolactam-polyvinyl acetate-polyethylene glycol graft | 5-65 |
| Mannitol or dibasic calcium phosphate | 5-65 |
| Crosscarmellose sodium or Sodium starch glycolate | 0.5-10 |
| Colloidal silicon dioxide | 0.25-2 |
| Magnesium stearate or Sodium stearyl fumarate | 0.25-2 |

| ***Film coating*** | |
|---|---|
| Opadry AMB or Collicoat IR | 1-5 |

Production Method: Valsartan, amlodipine besylate, polyvinyl caprolactam- polyvinyl acetate-polyethylene glycol graft copolymer, mannitole and crosscarmellose sodium are mixed, extruded by being melted together, cooled and sieved. Then, colloidal silicone dioxide is added to the obtained granule. Finally, magnesium stearate is added and mixed. Compression is applied in the tablet machine or filled into capsule. The coating process is realized with a coating material such as Opadry AMB or Collicoat IR having moisture barrier.

### c.

| **Ingredients** | **% Weight** |
|---|---|
| ***Core tablet*** | |
| Valsartan | 5-40 |
| Amlodipine Besylate | 0.3-1.3 |
| Hydrochlorothiazide | 0.5-3.5 |
| Polyvinyl caprolactam-polyvinyl acetate-polyethylene glycol graft | 5-60 |
| Mannitol or dibasic calcium phosphate | 5-60 |
| Crosscarmellose sodium or Sodium starch glycolate | 0.5-10 |
| Colloidal silicon dioxide | 0.25-2 |
| Magnesium stearate or Sodium stearyl fumarate | 0.25-2 |

| ***Film coating*** | |
|---|---|
| Opadry AMB or Collicoat IR | 1-5 |

**Production Method:** Valsartan, amlodipine besylate, hydrochlorothiazide, polyvinyl caprolactam- polyvinyl acetate-polyethylene glycol graft copolymer, mannitole and crosscarmellose sodium are mixed, extruded by being melted together, cooled and sieved. Then, colloidal silicone dioxide is added to the obtained granule. Finally, magnesium stearate is added and mixed. Compression is applied in the tablet machine or filled into capsule. The coating process is realized with a coating material such as Opadry AMB or Collicoat IR having moisture barrier.

### d.

| **Ingredients** | **% Weight (w/w)** |
|---|---|
| **Core tablet** | |
| *1^{st} Layer* | |
| Valsartan | 5-40 |
| Polyvinyl caprolactam-polyvinyl acetate-polyethylene glycol graft | 5-30 |
| Mannitol or dibasic calcium phosphate | 2.5-30 |
| Crosscarmellose sodium or Sodium starch glycolate | 0.5-10 |
| Colloidal silicon dioxide | 0.125-2.0 |

| *2^{nd} Layer* | |
|---|---|
| Amlodipine Besylate | 0.3-1.3 |
| Hydrochlorothiazide | 0.5-3.5 |
| Polyvinyl caprolactam-polyvinyl acetate-polyethylene glycol graft | 5-30 |
| Mannitol or dibasic calcium phosphate | 2.5-30 |
| Magnesium stearate or Sodium stearyl fumarate | 0.125-2.0 |
| **Film coating** | |
| Opadry AMB or Collicoat IR | 1-5 |

**Production Method (1) (Wet granulation + Hot melting):** In the 1^{st} layer valsartan is sieved with colloidal silicone dioxide and mixed (co-shifting). Afterwards, crosscarmellose sodium and mannitole are added to this mixture and blended. Afterwards, this mixture is granulated, dried and sieved with the alcoholic/hydroalcoholic solution of polyvinyl caprolactam-polyvinyl acetate-polyethylene glycol graft copolymer. Afterwards, it is mixed with magnesium stearate.

In the 2^{nd} layer amlodipine besylate, hydrochlorothiazide, mannitol and polyvinyl caprolactam- polyvinyl acetate-polyethylene glycol graft copolymer are mixed, extruded by being melted together, cooled and sieved. It is mixed with magnesium stearate. Afterwards, powders prepared for two layers are compressed in the bi layer tablet machine or may be filled into capsules. The coating process is realized with a coating material such as Opadry AMB or Collicoat IR having moisture barrier.

**Production Method (2) (Hot melting + Wet granulation):** In the 1^{st} layer valsartan, colloidal silicone dioxide, crosscarmellose sodium, mannitol and polyvinyl caprolactam- polyvinyl acetate-polyethylene glycol graft copolymer are mixed, extruded by being melted together, cooled and sieved. Afterwards, it is mixed with magnesium stearate.

In the 2^{nd} layer amlodipine besylate, hydrochlorothiazide, mannitole are mixed. The mixture is granulated, dried and sieved with the alcoholic/hydroalcoholic solution of polyvinyl caprolactam-polyvinyl acetate-polyethylene glycol graft copolymer. It is mixed with magnesium stearate. Afterwards, powders prepared for two layers are compressed in the bi layer tablet machine or may be filled into capsules. The coating process is realized with a coating material such as Opadry AMB or Collicoat IR having moisture barrier.

### e.

| **Ingredients** | **% Weight** |
|---|---|
| **Core tablet** | |
| *1st Layer* | |
| Valsartan | 5-40 |
| Polyvinyl caprolactam-polyvinyl acetate-polyethylene glycol graft | 3-20 |
| Crosscarmellose sodium or Sodium starch glycolate | 0.5-10 |
| Mannitol or dibasic calcium phosphate | 1-20 |
| Colloidal silicon dioxide | 0.25-3 |
| Magnesium stearate or Sodium stearyl fumarate | 0.1-0.5 |

| *2^{nd} Layer* | |
|---|---|
| Amlodipine Besylate | 0.3-1.3 |
| Polyvinyl caprolactam-polyvinyl acetate-polyethylene glycol graft | 3-20 |
| Mannitol or dibasic calcium phosphate | 1-20 |
| Magnesium stearate or Sodium stearyl fumarate | 0.1-0.5 |

| *3nd Layer* | |
|---|---|
| Hydrochlorothiazide | 0.5-3.5 |
| Polyvinyl caprolactam-polyvinyl acetate-polyethylene glycol graft | 3-20 |
| Mannitol or dibasic calcium phosphate | 1-20 |
| Magnesium stearate or Sodium stearyl fumarate | 0.1-0.5 |

| **Film coating** | |
|---|---|
| Opadry AMB or Collicoat IR | 1-5 |

**Production Method:** In the 1^{st} layer valsartan, colloidal silicone dioxide, crosscarmellose sodium, mannitol and polyvinyl caprolactam- polyvinyl acetate-polyethylene glycol graft copolymer are mixed, extruded by being melted together, cooled and sieved. Afterwards, it is mixed with magnesium stearate.

In the 2^{nd} layer amlodipine besylate, mannitol and polyvinyl caprolactam- polyvinyl acetate-polyethylene glycol graft copolymer are mixed, extruded by being melted together, cooled and sieved. Mixed with magnesium stearate.

In the 3^{rd} layer hydrochlorothiazide, mannitol and polyvinyl caprolactam- polyvinyl acetate-polyethylene glycol graft copolymer are mixed, extruded by being melted together, cooled and sieved. It is mixed with magnesium stearate. Afterwards, powders prepared for three layers are compressed in the tri layer tablet machine or may be filled into capsules. The coating process is realized with a coating material such as Opadry AMB or Collicoat IR having moisture barrier.

In the hot melting methods mentioned above, it is possible to add suitable plasticizers into the mixtures.

### D. DRUG LAYERING

| **Ingredients** | **% Weight** |
|---|---|
| Valsartan | 5-40 |
| Amlodipine Besylate | 0.3-1.3 |
| Hydrochlorothiazide | 0.5-3.5 |
| Polyvinyl caprolactam-polyvinyl acetate-polyethylene glycol graft | 5-60 |
| 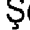 eker veya Mikrokristalin selüloz nötral pellet | 5-90 |
| Mannitol or dibasic calcium phosphate | 5-60 |
| Crosscarmellose sodium or Sodium starch glycolate | 0.5-10 |
| Magnesium stearate or Sodium stearyl fumarate | 0.25-2.0 |

| ***Film coating*** | |
|---|---|
| Opadry AMB or Collicoat IR | 1-5 |

Production Method: The alcoholic solution of valsartan, amlodipine besylate, hydrochlorothiazide and polyvinyl caprolactam- polyvinyl acetate- polyethylene glycole graft copolymer is prepared and sugar neutral pellets are layered in the fluidized bed system with this solution. The obtained pellets are first mixed with mannitole and crosscarmellose sodium and mixed with magnesium stearate for a short time and then, compressed in a tablet compression machine or filled into capsules. The coating process is realized with a coating material such as Opadry AMB or Collicoat IR having moisture barrier.

### E. SPRAY DRYING

| **Ingredients** | **% Weight** |
|---|---|
| Valsartan | 5-40 |
| Amlodipine Besylate | 0.3-1.3 |
| Hydrochlorothiazide | 0.5-3.5 |
| Polyvinyl caprolactam-polyvinyl acetate-polyethylene glycol graft | 5-60 |
| Mannitol or dibasic calcium phosphate | 5-60 |
| Crosscarmellose sodium or Sodium starch glycolate | 0.5-10 |
| Magnesium stearate or Sodium stearyl fumarate | 0.25-2.0 |

| ***Film coating*** | |
|---|---|
| Opadry AMB or Collicoat IR | 1-5 |

**Production Method:** The alcoholic solution of valsartan, amlodipine besylate, hydrochlorothiazide and polyvinyl caprolactam- polyvinyl acetate- polyethylene glycole graft copolymer is prepared and this solution is granulated by spraying to spray drier. The obtained granules are mixed with mannitole and crosscarmellose sodium and mixed with magnesium stearate for a short time and then, compressed in a tablet compression machine or filled into capsules. The coating process is realized with a coating material such as Opadry AMB or Collicoat IR having moisture barrier.

### Examples - 2

### A. DIRECT COMPRESSION

### a.

| **Ingredients** | **% Weight** |
|---|---|
| ***Core tablet*** | |
| Azilsartan Medoxomil | 1-50 |
| Polyvinyl caprolactam-polyvinyl acetate-polyethylene glycol graft | 5-70 |
| Mannitol or dibasic calcium phosphate | 5-70 |
| Crosscarmellose sodium or Sodium starch glycolate | 0.5-10 |
| Colloidal silicon dioxide | 0.25-2 |
| Magnesium stearate or Sodium stearyl fumarate | 0.25-2 |

| ***Film coating*** | |
|---|---|
| Opadry AMB or Collicoat IR | 1-5 |

Production Method: Azilsartan medoxomil and colloidal silicone dioxide are sieved and mixed together (co-shifting). Afterwards, polyvinyl caprolactam-polyvinyl acetate-polyethylene glycol graft copolymer, crosscarmellose sodium and mannitol are added to this mixture and blended. Afterwards, this mixture is blended with magnesium stearate and compressed in the tablet machine or may be filled into capsules. The coating process is realized with a coating material such as Opadry AMB or Collicoat IR having moisture barrier.

### b.

| **Ingredients** | **% Weight** |
|---|---|
| ***Core tablet*** | |
| Azilsartan Medoxomil | 1-50 |
| Amlodipin besilat | 0.3-1.3 |
| Polyvinyl caprolactam-polyvinyl acetate-polyethylene glycol graft | 5-65 |
| Mannitol or dibasic calcium phosphate | 5-65 |
| Crosscarmellose sodium or Sodium starch glycolate | 0.5-10 |
| Colloidal silicon dioxide | 0.25-2 |
| Magnesium stearate or Sodium stearyl fumarate | 0.25-2 |

| ***Film coating*** | |
|---|---|
| Opadry AMB or Collicoat IR | 1-5 |

Production Method: Azilsartan Medoxomil and amlodipine besilate is sieved with colloidal silicone dioxide and mixed (co-shifting). Afterwards, polyvinyl caprolactam-polyvinyl acetate-polyethylene glycol graft copolymer, crosscarmellose sodium and mannitol are added to this mixture and blended. Afterwards, this mixture is blended with magnesium stearate and compressed in the tablet machine or may be filled into capsules. The coating process is realized with a coating material such as Opadry AMB or Collicoat IR having moisture barrier.

### c.

| **Ingredients** | **% Weight** |
|---|---|
| ***Core tablet*** | |
| Azilsartan Medoxomil | 1-50 |
| Amlodipin Besilat | 0.3-1.3 |
| Hydrochlorothiazide | 0.5-3.5 |
| Polyvinyl caprolactam-polyvinyl acetate-polyethylene glycol graft | 5-60 |
| Mannitol or dibasic calcium phosphate | 5-60 |
| Crosscarmellose sodium or Sodium starch glycolate | 0.5-10 |
| Colloidal silicon dioxide | 0.25-2 |
| Magnesium stearate or Sodium stearyl fumarate | 0.25-2 |

| ***Film coating*** | |
|---|---|
| Opadry AMB or Collicoat IR | 1-5 |

**Production Method:** Azilsartan Medoxomil, amlodipine besilate and hydrochlorothiazide are sieved with colloidal silicone dioxide and mixed (co-shifting). Afterwards, polyvinyl caprolactam-polyvinyl acetate-polyethylene glycol graft copolymer, crosscarmellose sodium and mannitol are added to this mixture and blended. Afterwards, this mixture is blended with magnesium stearate and compressed in the tablet machine or may be filled into capsules. The coating process is realized with a coating material such as Opadry AMB or Collicoat IR having moisture barrier.

*Co-shifting:* When the active agents and silicone dioxide are added by being sieved together, the fluidity of the powders improves.

### d.

| **Ingredients** | **% Weight (w/w)** |
|---|---|
| **Core tablet** | |

| *1st layer* | |
|---|---|
| Azilsartan Medoxomil | 1-50 |
| Polyvinyl caprolactam-polyvinyl acetate-polyethylene glycol graft | 5-30 |
| Mannitol or dibasic calcium phosphate | 2.5-30 |
| Crosscarmellose sodium or Sodium starch glycolate | 0.5-10 |
| Colloidal silicon dioxide | 0.125-1.5 |
| Magnesium stearate or Sodium stearyl fumarate | 0.125-2.0 |

| *2nd Layer* | |
|---|---|
| Amlodipin Besilat | 0.3-1.3 |
| Hydrochlorothiazide | 0.5-3.5 |
| Polyvinyl caprolactam-polyvinyl acetate-polyethylene glycol graft | 5-30 |
| Mannitol or dibasic calcium phosphate | 2.5-30 |
| Colloidal silicon dioxide | 0.125-1.5 |
| Magnesium stearate or Sodium stearyl fumarate | 0.125-2.0 |

| **Film coating** | |
|---|---|
| Opadry AMB or Collicoat IR | 1-5 |

**Production Method:** In the 1^{st} layer Azilsartan Medoxomil is sieved with colloidal silicone dioxide and mixed (co-shifting). Afterwards, polyvinyl caprolactam-polyvinyl acetate-polyethylene glycol graft copolymer, crosscarmellose sodium and mannitol are added to this mixture and blended. Afterwards, this mixture is blended with magnesium stearate. In the 2^{nd} layer, in a different mixer, amlodipine besilate and hydrochlorothiazide is sieved with colloidal silicone dioxide and mixed (co-shifting). Afterwards, polyvinyl caprolactam-polyvinyl acetate-polyethylene glycol graft copolymer and mannitol are added to this mixture and blended. Afterwards, this mixture is is blended with magnesium stearate. Afterwards, powders prepared for two layers are compressed in the bi layer tablet machine or may be filled into capsules. The coating process is realized with a coating material such as Opadry AMB or Collicoat IR having moisture barrier.

### e.

| **Ingredients** | **% Weight (w/w)** |
|---|---|
| **Core tablet** | |
| *1st Layer* | |
| Azilsartan Medoxomil | 1-50 |
| Polyvinyl caprolactam-polyvinyl acetate-polyethylene glycol graft | 3-20 |
| Crosscarmellose sodium or Sodium starch glycolate | 0.25-5 |
| Mannitol or dibasic calcium phosphate | 1-20 |
| Colloidal silicon dioxide | 0.1-1 |
| Magnesium stearate or Sodium stearyl fumarate | 0.1-0.5 |

| *2nd layer* | |
|---|---|
| Amlodipin Besilat | 0.3-1.3 |
| Crosscarmellose sodium or Sodium starch glycolate | 0.25-5 |
| Polyvinyl caprolactam-polyvinyl acetate-polyethylene glycol graft | 3-20 |
| Mannitol or dibasic calcium phosphate | 1-20 |
| Colloidal silicon dioxide | 0.1-1 |
| Magnesium stearate or Sodium stearyl fumarate | 0.1-0.5 |

| *3rd Layer* | |
|---|---|
| Hydrochlorothiazide | 0.5-3.5 |
| Polyvinyl caprolactam-polyvinyl acetate-polyethylene glycol graft | 3-20 |
| Mannitol or dibasic calcium phosphate | 1-20 |
| Colloidal silicon dioxide | 0.1-1 |
| Magnesium stearate or Sodium stearyl fumarate | 0.1-0.5 |

| **Film coating** | |
|---|---|
| Opadry AMB or Collicoat IR | 1-5 |

**Production Method:** In the 1^{st} layer Azilsartan Medoxomil is sieved with colloidal silicone dioxide and mixed (co-shifting). Afterwards, polyvinyl caprolactam-polyvinyl acetate-polyethylene glycol graft copolymer, crosscarmellose sodium and mannitol are added to this mixture and blended. Afterwards, this mixture is blended with magnesium stearate. In the 2^{nd} layer, in a different mixer, amlodipine besilate is sieved with colloidal silicone dioxide and mixed (co-shifting). Afterwards, polyvinyl caprolactam-polyvinyl acetate-polyethylene glycol graft copolymer, crosscarmellose sodium and mannitol are added to this mixture and blended. Afterwards, this mixture is is blended with magnesium stearate. In the 3^{rd} layer, in a different mixer, hydrochlorothiazide is sieved with colloidal silicone dioxide and mixed (co-shifting). Afterwards, polyvinyl caprolactam-polyvinyl acetate-polyethylene glycol graft copolymer and mannitol are added to this mixture and blended. Afterwards, this mixture is is blended with magnesium stearate. Afterwards, powders prepared for three layers are compressed in the tri layer tablet machine or may be filled into capsules. The coating process is realized with a coating material such as Opadry AMB or Collicoat IR having moisture barrier.

### Examples - 3

### A. DIRECT COMPRESSION

### a.

| **Ingredients** | **% Weight** |
|---|---|
| ***Core tablet*** | |
| Olmesartan | 1-50 |
| Polyvinyl caprolactam-polyvinyl acetate-polyethylene glycol graft | 5-70 |
| Mannitol or dibasic calcium phosphate | 5-70 |
| Crosscarmellose sodium or Sodium starch glycolate | 0.5-10 |
| Colloidal silicon dioxide | 0.25-2 |
| Magnesium stearate or Sodium stearyl fumarate | 0.25-2 |

| ***Film coating*** | |
|---|---|
| Opadry AMB or Collicoat IR | 1-5 |

Production Method: Olmesartan and colloidal silicone dioxide are sieved and mixed together (co-shifting). Afterwards, polyvinyl caprolactam-polyvinyl acetate-polyethylene glycol graft copolymer, crosscarmellose sodium and mannitol are added to this mixture and blended. Afterwards, this mixture is blended with magnesium stearate and compressed in the tablet machine or may be filled into capsules. The coating process is realized with a coating material such as Opadry AMB or Collicoat IR having moisture barrier.

### b.

| **Ingredients** | **% Weight** |
|---|---|
| ***Core tablet*** | |
| Olmesartan | 1-50 |
| Amlodipin besilat | 0.3-1.3 |
| Polyvinyl caprolactam-polyvinyl acetate-polyethylene glycol graft | 5-65 |
| Mannitol or dibasic calcium phosphate | 5-65 |
| Crosscarmellose sodium or Sodium starch glycolate | 0.5-10 |
| Colloidal silicon dioxide | 0.25-2 |
| Magnesium stearate or Sodium stearyl fumarate | 0.25-2 |

| ***Film coating*** | |
|---|---|
| Opadry AMB or Collicoat IR | 1-5 |

Production Method: Olmesartan and amlodipine besilate is sieved with colloidal silicone dioxide and mixed (co-shifting). Afterwards, polyvinyl caprolactam-polyvinyl acetate-polyethylene glycol graft copolymer, crosscarmellose sodium and mannitol are added to this mixture and blended. Afterwards, this mixture is blended with magnesium stearate and compressed in the tablet machine or may be filled into capsules. The coating process is realized with a coating material such as Opadry AMB or Collicoat IR having moisture barrier.

### c.

| **Ingredients** | **% Weight** |
|---|---|
| ***Core tablet*** | |
| Olmesartan | 1-50 |
| Amlodipin Besilat | 0.3-1.3 |
| Hydrochlorothiazide | 0.5-3.5 |
| Polyvinyl caprolactam-polyvinyl acetate-polyethylene glycol graft | 5-60 |
| Mannitol or dibasic calcium phosphate | 5-60 |
| Crosscarmellose sodium or Sodium starch glycolate | 0.5-10 |
| Colloidal silicon dioxide | 0.25-2 |
| Magnesium stearate or Sodium stearyl fumarate | 0.25-2 |

| ***Film coating*** | |
|---|---|
| Opadry AMB or Collicoat IR | 1-5 |

**Production Method:** Olmesartan, amlodipine besilate and hydrochlorothiazide are sieved with colloidal silicone dioxide and mixed (co-shifting). Afterwards, polyvinyl caprolactam-polyvinyl acetate-polyethylene glycol graft copolymer, crosscarmellose sodium and mannitol are added to this mixture and blended. Afterwards, this mixture is blended with magnesium stearate and compressed in the tablet machine or may be filled into capsules. The coating process is realized with a coating material such as Opadry AMB or Collicoat IR having moisture barrier.

*Co-shifting:* When the active agents and silicone dioxide are added by being sieved together, the fluidity of the powders improves.

### d.

| | |
|---|---|
| **Ingredients** | **% Weight** |
| **Core tablet** | |
| *1st layer* | |
| Olmesartan | 1-50 |
| Polyvinyl caprolactam-polyvinyl acetate-polyethylene glycol graft | 5-30 |
| Mannitol or dibasic calcium phosphate | 2.5-30 |
| Crosscarmellose sodium or Sodium starch glycolate | 0.5-10 |
| Colloidal silicon dioxide | 0.125-1.5 |
| Magnesium stearate or Sodium stearyl fumarate | 0.125-2.0 |

| *2nd Layer* | |
|---|---|
| Amlodipin Besilat | 0.3-1.3 |
| Hydrochlorothiazide | 0.5-3.5 |
| Polyvinyl caprolactam-polyvinyl acetate-polyethylene glycol graft | 5-30 |
| Mannitol or dibasic calcium phosphate | 2.5-30 |
| Colloidal silicon dioxide | 0.125-1.5 |
| Magnesium stearate or Sodium stearyl fumarate | 0.125-2.0 |

| **Film coating** | |
|---|---|
| Opadry AMB or Collicoat IR | 1-5 |

**Production Method:** In the 1^{st} layer olmesartan is sieved with colloidal silicone dioxide and mixed (co-shifting). Afterwards, polyvinyl caprolactam-polyvinyl acetate-polyethylene glycol graft copolymer, crosscarmellose sodium and mannitol are added to this mixture and blended. Afterwards, this mixture is blended with magnesium stearate. In the 2^{nd} layer, in a different mixer, amlodipine besilate and hydrochlorothiazide is sieved with colloidal silicone dioxide and mixed (co-shifting). Afterwards, polyvinyl caprolactam-polyvinyl acetate-polyethylene glycol graft copolymer and mannitol are added to this mixture and blended. Afterwards, this mixture is is blended with magnesium stearate. Afterwards, powders prepared for two layers are compressed in the bi layer tablet machine or may be filled into capsules. The coating process is realized with a coating material such as Opadry AMB or Collicoat IR having moisture barrier.

### e.

| **Ingredients** | **% Weight (w/w)** |
|---|---|
| **Core tablet** | |
| *1st Layer* | |
| Olmesartan | 1-50 |
| Polyvinyl caprolactam-polyvinyl acetate-polyethylene glycol graft | 3-20 |
| Crosscarmellose sodium or Sodium starch glycolate | 0.25-5 |
| Mannitol or dibasic calcium phosphate | 1-20 |
| Colloidal silicon dioxide | 0.1-1 |
| Magnesium stearate or Sodium stearyl fumarate | 0.1-0.5 |

| *2nd layer* | |
|---|---|
| Amlodipin Besilat | 0.3-1.3 |
| Crosscarmellose sodium or Sodium starch glycolate | 0.25-5 |
| Polyvinyl caprolactam-polyvinyl acetate-polyethylene glycol graft | 3-20 |
| Mannitol or dibasic calcium phosphate | 1-20 |
| Colloidal silicon dioxide | 0.1-1 |
| Magnesium stearate or Sodium stearyl fumarate | 0.1-0.5 |

| *3rd Layer* | |
|---|---|
| Hydrochlorothiazide | 0.5-3.5 |
| Polyvinyl caprolactam-polyvinyl acetate-polyethylene glycol graft | 3-20 |
| Mannitol or dibasic calcium phosphate | 1-20 |
| Colloidal silicon dioxide | 0.1-1 |
| Magnesium stearate or Sodium stearyl fumarate | 0.1-0.5 |

| **Film coating** | |
|---|---|
| Opadry AMB or Collicoat IR | 1-5 |

**Production Method:** In the 1^{st} layer olmesartan is sieved with colloidal silicone dioxide and mixed (co-shifting). Afterwards, polyvinyl caprolactam-polyvinyl acetate-polyethylene glycol graft copolymer, crosscarmellose sodium and mannitol are added to this mixture and blended. Afterwards, this mixture is blended with magnesium stearate. In the 2^{nd} layer, in a different mixer, amlodipine besilate is sieved with colloidal silicone dioxide and mixed (co-shifting). Afterwards, polyvinyl caprolactam-polyvinyl acetate-polyethylene glycol graft copolymer, crosscarmellose sodium and mannitol are added to this mixture and blended. Afterwards, this mixture is is blended with magnesium stearate. In the 3^{rd} layer, in a different mixer, hydrochlorothiazide is sieved with colloidal silicone dioxide and mixed (co-shifting). Afterwards, polyvinyl caprolactam-polyvinyl acetate-polyethylene glycol graft copolymer and mannitol are added to this mixture and blended. Afterwards, this mixture is is blended with magnesium stearate. Afterwards, powders prepared for three layers are compressed in the tri layer tablet machine or may be filled into capsules. The coating process is realized with a coating material such as Opadry AMB or Collicoat IR having moisture barrier.

### Examples - 4

### A. DIRECT COMPRESSION

### a.

| **Ingredients** | **% Weight** |
|---|---|
| ***Core tablet*** | |
| Eprosartan | 40-90 |
| Polyvinyl caprolactam-polyvinyl acetate-polyethylene glycol graft | 5-70 |
| Mannitol or dibasic calcium phosphate | 5-70 |
| Crosscarmellose sodium or Sodium starch glycolate | 0.5-10 |
| Colloidal silicon dioxide | 0.25-2 |
| Magnesium stearate or Sodium stearyl fumarate | 0.25-2 |

| ***Film coating*** | |
|---|---|
| Opadry AMB or Collicoat IR | 1-5 |

Production Method: Eprosartan and colloidal silicone dioxide are sieved and mixed together (co-shifting). Afterwards, polyvinyl caprolactam-polyvinyl acetate-polyethylene glycol graft copolymer, crosscarmellose sodium and mannitol are added to this mixture and blended. Afterwards, this mixture is blended with magnesium stearate and compressed in the tablet machine or may be filled into capsules. The coating process is realized with a coating material such as Opadry AMB or Collicoat IR having moisture barrier.

### b.

| **Ingredients** | **% Weight** |
|---|---|
| ***Core tablet*** | |
| Eprosartan | 40-90 |
| Amlodipin besilat | 0.3-1.3 |
| Polyvinyl caprolactam-polyvinyl acetate-polyethylene glycol graft | 5-65 |
| Mannitol or dibasic calcium phosphate | 5-65 |
| Crosscarmellose sodium or Sodium starch glycolate | 0.5-10 |
| Colloidal silicon dioxide | 0.25-2 |
| Magnesium stearate or Sodium stearyl fumarate | 0.25-2 |

| ***Film coating*** | |
|---|---|
| Opadry AMB or Collicoat IR | 1-5 |

Production Method: Eprosartan and amlodipine besilate is sieved with colloidal silicone dioxide and mixed (co-shifting). Afterwards, polyvinyl caprolactam-polyvinyl acetate-polyethylene glycol graft copolymer, crosscarmellose sodium and mannitol are added to this mixture and blended. Afterwards, this mixture is blended with magnesium stearate and compressed in the tablet machine or may be filled into capsules. The coating process is realized with a coating material such as Opadry AMB or Collicoat IR having moisture barrier.

### c.

| **Ingredients** | **% Weight** |
|---|---|
| ***Core tablet*** | |
| Eprosartan | 40-90 |
| Amlodipin Besilat | 0.3-1.3 |
| Hydrochlorothiazide | 0.5-3.5 |
| Polyvinyl caprolactam-polyvinyl acetate-polyethylene glycol graft | 5-60 |
| Mannitol or dibasic calcium phosphate | 5-60 |
| Crosscarmellose sodium or Sodium starch glycolate | 0.5-10 |
| Colloidal silicon dioxide | 0.25-2 |
| Magnesium stearate or Sodium stearyl fumarate | 0.25-2 |

| ***Film coating*** | |
|---|---|
| Opadry AMB or Collicoat IR | 1-5 |

**Production Method:** Eprosartan, amlodipine besilate and hydrochlorothiazide are sieved with colloidal silicone dioxide and mixed (co-shifting). Afterwards, polyvinyl caprolactam-polyvinyl acetate-polyethylene glycol graft copolymer, crosscarmellose sodium and mannitol are added to this mixture and blended. Afterwards, this mixture is blended with magnesium stearate and compressed in the tablet machine or may be filled into capsules. The coating process is realized with a coating material such as Opadry AMB or Collicoat IR having moisture barrier.

*Co-shifting:* When the active agents and silicone dioxide are added by being sieved together, the fluidity of the powders improves.

### d.

| **Ingredients** | **% Weight (w/w)** |
|---|---|
| **Core tablet** | |
| *1st layer* | |
| Eprosartan | 40-90 |
| Polyvinyl caprolactam-polyvinyl acetate-polyethylene glycol graft | 5-30 |
| Mannitol or dibasic calcium phosphate | 2.5-30 |
| Crosscarmellose sodium or Sodium starch glycolate | 0.5-10 |
| Colloidal silicon dioxide | 0.125-1.5 |
| Magnesium stearate or Sodium stearyl fumarate | 0.125-2.0 |

| *2nd Layer* | |
|---|---|
| Amlodipin Besilat | 0.3-1.3 |
| Hydrochlorothiazide | 0.5-3.5 |
| Polyvinyl caprolactam-polyvinyl acetate-polyethylene glycol graft | 5-30 |
| Mannitol or dibasic calcium phosphate | 2.5-30 |
| Colloidal silicon dioxide | 0.125-1.5 |
| Magnesium stearate or Sodium stearyl fumarate | 0.125-2.0 |

| **Film coating** | |
|---|---|
| Opadry AMB or Collicoat IR | 1-5 |

**Production Method:** In the 1^{st} layer Eprosartan is sieved with colloidal silicone dioxide and mixed (co-shifting). Afterwards, polyvinyl caprolactam-polyvinyl acetate-polyethylene glycol graft copolymer, crosscarmellose sodium and mannitol are added to this mixture and blended. Afterwards, this mixture is blended with magnesium stearate. In the 2^{nd} layer, in a different mixer, amlodipine besilate and hydrochlorothiazide is sieved with colloidal silicone dioxide and mixed (co-shifting). Afterwards, polyvinyl caprolactam-polyvinyl acetate-polyethylene glycol graft copolymer and mannitol are added to this mixture and blended. Afterwards, this mixture is is blended with magnesium stearate. Afterwards, powders prepared for two layers are compressed in the bi layer tablet machine or may be filled into capsules. The coating process is realized with a coating material such as Opadry AMB or Collicoat IR having moisture barrier.

### e.

| **Ingredients** | **% Weight (w/w)** |
|---|---|
| **Core tablet** | |
| *1st Layer* | |
| Eprosartan | 40-90 |
| Polyvinyl caprolactam-polyvinyl acetate-polyethylene glycol graft | 3-20 |
| Crosscarmellose sodium or Sodium starch glycolate | 0.25-5 |
| Mannitol or dibasic calcium phosphate | 1-20 |
| Colloidal silicon dioxide | 0.1-1 |
| Magnesium stearate or Sodium stearyl fumarate | 0.1-0.5 |

| *2nd layer* | |
|---|---|
| Amlodipin Besilat | 0.3-1.3 |
| Crosscarmellose sodium or Sodium starch glycolate | 0.25-5 |
| Polyvinyl caprolactam-polyvinyl acetate-polyethylene glycol graft | 3-20 |
| Mannitol or dibasic calcium phosphate | 1-20 |
| Colloidal silicon dioxide | 0.1-1 |
| Magnesium stearate or Sodium stearyl fumarate | 0.1-0.5 |

| *3rd Layer* | |
|---|---|
| Hydrochlorothiazide | 0.5-3.5 |
| Polyvinyl caprolactam-polyvinyl acetate-polyethylene glycol graft | 3-20 |
| Mannitol or dibasic calcium phosphate | 1-20 |
| Colloidal silicon dioxide | 0.1-1 |
| Magnesium stearate or Sodium stearyl fumarate | 0.1-0.5 |

| **Film coating** | |
|---|---|
| Opadry AMB or Collicoat IR | 1-5 |

**Production Method:** In the 1^{st} layer Eprosartan is sieved with colloidal silicone dioxide and mixed (co-shifting). Afterwards, polyvinyl caprolactam-polyvinyl acetate-polyethylene glycol graft copolymer, crosscarmellose sodium and mannitol are added to this mixture and blended. Afterwards, this mixture is blended with magnesium stearate. In the 2^{nd} layer, in a different mixer, amlodipine besilate is sieved with colloidal silicone dioxide and mixed (co-shifting). Afterwards, polyvinyl caprolactam-polyvinyl acetate-polyethylene glycol graft copolymer, crosscarmellose sodium and mannitol are added to this mixture and blended. Afterwards, this mixture is is blended with magnesium stearate. In the 3^{rd} layer, in a different mixer, hydrochlorothiazide is sieved with colloidal silicone dioxide and mixed (co-shifting). Afterwards, polyvinyl caprolactam-polyvinyl acetate-polyethylene glycol graft copolymer and mannitol are added to this mixture and blended. Afterwards, this mixture is is blended with magnesium stearate. Afterwards, powders prepared for three layers are compressed in the tri layer tablet machine or may be filled into capsules. The coating process is realized with a coating material such as Opadry AMB or Collicoat IR having moisture barrier.

Without being limited to the examples, valsartan, olmesartan, azilsartan, eprosartan, amlodipine and hydrochlorothiazide may be present in various composition formulations in an amount of 10, 20,80, 160, 300, 320, 600 mg, 5 to 10 mg, 6.25, 10, 12.5, 25, 50 mg respectively.

Surprisingly, stable compositions having high solubility and dissolution rate and content uniformity are obtained with the present invention. The said compositions comprises_angiotensin II receptor antagonist with polyvinyl caprolactam- polyvinyl acetate-polyethylene glycol graft copolymer. Additionally, calcium channel blocker and thiazide diuretic may be added to the compositions. A uniform composition is obtained by the virtue of the applied process. Besides that, the possible fluidity problems observed during production are prevented. The desired solubility and dissolution rates are realized. Additionally, the interaction between the active agents present in the bilayer and trilayer compositions decreases and more stable compositions are obtained.

Polymers with low glass transition temperature and melting point are used when hot melting method is preferred in the composition. In addition to this, the stability of the active agent is increased by using plasticizer agents which decrease the glass transition temperature. The plasticizer used in the hot-melting process decreases the glass transition temperature of the polymers used in hot-melting and thus, it makes it possible to formulate the active agent in lower temperatures. Eventually, the products becomes more stable.

Additionally, the pharmaceutical compositions of the present invention may contain one or more pharmaceutically acceptable excipients. Pharmaceutically acceptable suitable excipients include, but are not limited to filling agents, glidants, lubricants, dispersing agents, surface active agents and the like and the mixtures thereof.

The present invention is used for preventing or treating hypertension in mammals, particularly in humans.

The term "composition" means the formulation whereas it also means the collective use of the formulation and the package or the blister in which the formulation is stored.

It is also possible to use the following additional excipients in the formulation.

As lubricant, the formulation may include, but not limited to sodium stearyl fumarate, polyethylene glycol, stearic acid, metal stearates, boric acid, sodium chloride benzoate and acetate, sodium or magnesium lauryl sulfate or mixtures thereof.

The formulation may include -but not limited to- methyl paraben and propyl paraben and salts thereof (e.g. sodium or potassium salts), sodium benzoate, citric acid, benzoic acid, butylated hydroxytoluene and butylated hydroxyanisole or mixtures thereof, as preservative.

Suitable surface active agents, may include but not limited to sodium lauryl sulfate, dioctyl sulfosuccinate, polysorbates and polyoxyethylene alkyl esters and ethers thereof, glyceryl monolaurate saponins, sorbitan laurate, sodium lauryl sulfate, magnesium lauryl sulfate or mixtures thereof.

This invention is defined with the reference of the examples given above. Although the examples do not limit the object of the present invention, it must be evaluated in the light of detailed explanations stated above.

Salt of amlodipine besylate may be preferred in the formulation.

## Claims

1. The invention is related to a pharmaceutical composition, **characterized in that**; it comprises angiotensin II receptor antagonist and polyvinyl caprolactam-polyvinyl acetate-polyethylene glycol graft copolymer.

2. A pharmaceutical composition according to Claim 1, wherein, the said angiotension II receptor antagonist is selected among valsartan, losartan, azilsartan, irbesartan, telmisartan, candesartan, eprosartan, olmesartan medoxomil.

3. A pharmaceutical composition according to any one of the preceding claims, wherein, the said angiotension II receptor antagonist comprises valsartan or a pharmaceutically acceptable salt or ester or racemic mixture thereof.

4. A pharmaceutical composition according to any one of the preceding claims, wherein, it additionally comprises a calcium channel blocker selected from amlodipine, felodipine, azelnidipine, bepridil, diltiazem, niphedipin, verapamil.

5. A pharmaceutical composition according to any one of the preceding claims, wherein, the said calcium channel blocker comprises amlodipine or a pharmaceutically acceptable salt or ester or racemic mixture thereof.

6. A pharmaceutical composition according to any one of the preceding claims, wherein, it additionally comprises a thiazide diuretic selected from hydrochlorothiazide, chlorinetalidone, indapamide, flupamide.

7. A pharmaceutical composition according to any one of the preceding claims, wherein, the said thiazide diuretic comprises hydrochlorothiazide or a pharmaceutically acceptable salt or ester or racemic mixture thereof.

8. A pharmaceutical composition according to any one of the preceeding claims, wherein, the said composition is tablet.

9. A pharmaceutical composition according to any one of the preceding claims, wherein, the said tablet is compressed with a pressure of 20 to 50 kN, preferably 25 to 45 kN, and more preferably 30 to 37 kN.

10. A pharmaceutical composition according to any one of the preceding claims, wherein, it additionally comprises at least one excipient.

11. A pharmaceutical composition according to any one of the preceding claims, wherein, the said excipient is at least one of the substances selected from the group consisting of diluents, binders, plasticizers, disintegrants, glidants, lubricants or a mixture of them.

12. A pharmaceutical composition according to any one of the preceding claims, wherein, the said diluents are at least one of the substances selected from the group consisting of dextrose, magnesium carbonate, magnesium oxide, sucrose, cellulose acetate, maltodextrine, lactose, microcrystalline cellulose, starch, mannitole, calcium phosphate anhydrate, calcium phosphate dihydrate, calcium phosphate trihydrate, dibasic calcium phosphate, glucose or a mixture of them.

13. A pharmaceutical composition according to any one of the preceding claims, wherein, the binders are at least one of the substances selected from the group consisting of hydroxyethyl cellulose (HEC), chitosan, carbomer, Carragen, pectin, collagen, guar gum, gum tragacanth, shellac, carnauba wax, cetyl alcohol, stearic acid, polyamide, hydrogenated castor oil, alginic acid, beeswax, methacrylic acid/ethyl acrylate copolymer, dimethyl amino ethyl methacrylate/butyl methacrylate/metal methacrylate copolymer, hydroxypropyl methyl cellulose acetate succinate, gelatine, derivatives of methacrylate, paraffine, polyvinyl acetate, cellulose acetate, cellulose phthalate, cellulose propionate, cellulose butyrate, setostearyl alcohol, polimethacrylate, glyceryl behenate, polyviniylpyrolidone (povidone), cellulose derivatives such as hydroxypropyl methyl cellulose (HPMC), hydroxypropyl cellulose (HPC), carboxy methyl cellulose (CMC), methyl cellulose (MC), ethyl cellulose, polyethylene oxide, gelatine or a mixture of them.

14. A pharmaceutical composition according to any one of the preceding claims, wherein the said plasticizer are preferably at least one of the substances selected from the group consisting of castor oil, glycerin, citrate esters (acetyl tri-n-butyl citrate, acetyl triethyl citrate, tri-n-butyl citrate, triethyl citrate) dibutyl sebacate, triacetin, diethyl phthalate, low molecular weight polyethylene glycols or a mixture of them in suitable amounts.

15. A pharmaceutical composition according to any one of the preceding claims, wherein, the disintegrants are at least one of the substances selected from the group consisting of crosscarmellose sodium, sodium starch glycolate, crospovidone, starch and alginic acid or a mixture of them.

16. A pharmaceutical composition according to any one of the preceding claims, wherein, the glidants are at least one of the substances selected from the group consisting of colloidal silicone dioxide, talc, aluminium silicate, magnesium silicate or a mixture of them.

17. A pharmaceutical composition according to any one of the preceding claims, wherein, the said lubricant is at least one of the substances selected from the group consisting of glyceryl behenate, glyceryl monostearate calcium stearate, magnesium stearate, mineral oil, sodium stearyl fumarate, talc, polyethylene glycol, stearic acid or a mixture of them.

18. A process for preparing a pharmaceutical composition according to any one of the preceding claims, wherein, it comprises the steps below:
a. adding colloidal silicone dioxide to valsartan alone or mixture of valsartan and amlodipine besylate or valsartan, amlodipine besylate and hydrochlorothiazide colloidal silicone dioxide and mixing them together by sieving,
b. adding polyvinyl caprolactam-polyvinyl acetate-polyethylene glycol graft copolymer, crosscarmellose sodium and mannitol to this mixture and blending it,
c. afterwards, adding magnesium stearate to this mixture and blending it,
d. compressing the last mixture in the tablet machine or filling it into the capsule,
e. coating the tablet with a coating material.

19. A process for preparing a pharmaceutical composition according to any one of the preceding claims, wherein, it comprises the steps below:
a. adding colloidal silicone dioxide to valsartan alone or mixture of valsartan and amlodipine besylate or valsartan, amlodipine besylate and hydrochlorothiazide colloidal silicone dioxide and mixing them together by sieving,
b. adding crosscarmellose sodium and mannitole to this mixture and blending them,
c. granulating, drying and sieving with the addition of alcoholic/hydroalcoholic solution of polyvinyl caprolactam-polyvinyl acetate-polyethylene glycol graft copolymer to the mixture formed,
d. afterwards, adding magnesium stearate to this mixture and blending it,
e. compressing the last mixture in the tablet machine or filling it into the capsule,
f. coating the tablet with a coating material.

20. The pharmaceutical composition according to any of the preceding claims for use in preventing or treating the hypertension in mammalians and particularly in humans.
